Europäisches Patentamt

European Patent Office

Office européen des brevets

(11) Veröffentlichungsnummer: **0 258 297**
**B1**

(12) **EUROPÄISCHE PATENTSCHRIFT**

(45) Veröffentlichungstag der Patentschrift: **18.04.90**

(51) Int. Cl.⁵: **C 07 C 69/757, C 07 C 67/56**

(21) Anmeldenummer: **87901048.6**

(22) Anmeldetag: **03.02.87**

(86) Internationale Anmeldenummer:
**PCT/EP87/00049**

(87) Internationale Veröffentlichungsnummer:
**WO 87/04704 13.08.87 Gazette 87/18**

(54) VERFAHREN ZUR ABTRENNUNG UND GEWINNUNG VON CHLOROGENSÄURE.

(30) Priorität: **06.02.86 DE 3603574**

(43) Veröffentlichungstag der Anmeldung:
**09.03.88 Patentblatt 88/10**

(45) Bekanntmachung des Hinweises auf die
Patenterteilung:
**18.04.90 Patentblatt 90/16**

(84) Benannte Vertragsstaaten:
**AT BE CH DE FR GB IT LI LU NL SE**

(56) Entgegenhaltungen:
**keine**

(73) Patentinhaber: **ERGO Forschungsgesellschaft
mbH
Luruper Chaussee 145
D-2000 Hamburg 50 (DE)**

(72) Erfinder: **KOPSCH, Reiner
Möwenring 7d
D-2000 Schenefeld (DE)**
Erfinder: **GÖSSWEIN, Claus, F.
Ellernbrook 20
D-2110 Buchholz 5 (DE)**
Erfinder: **LUTZ, Henning
Hagenwisch 2a
D-2083 Halstenbek (DE)**
Erfinder: **BALL, Michael
Hagenwisch 2c
D-2083 Halstenbek (DE)**
Erfinder: **HUBERT, Peter
Hasenkamp 21
D-2150 Buxtehude (DE)**

(74) Vertreter: **UEXKÜLL & STOLBERG
Patentanwälte
Beselerstrasse 4
D-2000 Hamburg 52 (DE)**

EP 0 258 297 B1

# EP 0 258 297 B1

**Beschreibung**

Die Erfindung betrifft ein vereinfachtes Verfahren zur Abtrennung und Gewinnung von Chlorogensäure durch Extraktion von geeigneten pflanzlichen Rohstoffen und Aufarbeitung des Extraktes.

K. Gorter, Liebigs Ann. 358, 327—348 (1908), K. Freudenberg, Ber. 53, 232—239 (1920) sowie W. Plücker und W. Keilholz, Z. Lebensmittelunters. u. Forsch. 66, 200—238 (1933) haben Verfahren zur Gewinnung von Chlorogensäure beschrieben. Bei allen bekannten Verfahren ist der erste Schritt die Gewinnung eines Kalium-Coffein-Chlorogenatkomplexes. Dieser wird nach unterschiedlichen Methoden gereinigt, z.B. durch mehrmaliges Umkristallisieren aus Ethanol/Wasser oder durch Fällen mit Bleiacetat. Das Abtrennen des Coffeins aus dem gereinigten Komplex kann beispielsweise durch Extraktion der wässrigen Lösung mit Chloroform erfolgen. Die Chlorogensäure wird anschließend durch Zusatz von Schwefelsäure gefällt und durch Umkristallisieren aus Wasser weiter gereinigt. Die Ausbeute an Chlorogensäure wird mit 1%, bezogen auf die eingesetzte Rohkaffeemenge, angegeben, was bezogen auf die im Rohkaffee enthaltene Menge an Chlorogensäure einer Ausbeute von ca. 20% entspricht.

Andere Verfahren zur Gewinnung von Chlorogensäure beruhen auf einer Modifikation oder Kombination der früheren Verfahren. So beschreibt beispielsweise U. Fiedler, Arzneimittelforschung 4, 41—45 (1954) ein Verfahren, welches eine Kombination der Methoden von Freudenberg sowie von Plücker und Keilholz darstellt. Danach werden grüne Kaffeebohnen getrocknet und zerkleinert und danach zunächst mit Petrolether und anschließend mit heißem Wasser extrahiert, bis die Extrakte keine Chlorogensäure mehr enthalten. Die vereinigten Auszüge werden eingeengt und mit Bariumacetat gefällt. Anschließend wird das Filtrat mit Schwefelsäure genau neutralisiert, wobei gleichzeitig das überschüssige Barium entfernt wird. Aus dem neutralen Filtrat wird mit Hilfe von Bleiacetat die Chlorogensäure als Komplex abgeschieden, welcher mit heißem Wasser gewaschen und anschließend nach Aufschlämmen in heißem Wasser mit Schwefelwasserstoff zersetzt wird. Aus dem eingeengten Filtrat scheidet sich nach zwei- bis dreitägigem Stehen im Kühlschrank der Kalium-Coffein-Chlorogenatkomplex ab. Aus dem Komplex wird das Coffein mit Chloroform entfernt und schließlich durch schwaches Ansäuern die freie Chlorogensäure erhalten.

In Chemical Abstracts 73, 32171E (1970) wird ein Verfahren wiedergegeben, nach dem die isomeren Chlorogensäuren durch Säulenchromatographie an Kieselsäuren und Elution mit einem Chloroform-Butanolgradienten eluiert werden. In Chemical Abstracts 89, 88927D (1978) ist die Extraktion von Chlorogensäure aus einem wässrigen Extrakt grüner Kaffeebohnen durch Anionaustauscher wie Dowex 44, Amberlite IRA 410 oder IRA 47 und Dowex 11 beschrieben.

Es ist deutlich, daß die vorbekannten Verfahren recht umständlich sind und keine hohen Ausbeuten liefern können. So weit Chlorogensäure im Handel erhältlich ist, liegt ihr Preis demgemäß auch extrem hoch. Darüber hinaus wird nur 3-Chlorogensäure bzw. das Isomerengemisch angeboten, ein brauchbares Verfahren zur wirtschaftlichen Gewinnung der 4- bzw. der 5-Chlorogensäure ist bislang nicht bekannt. Der Begriff 3-Chlorogensäure kennzeichnet die 3-Caffeoyl-Chinasäure und wird nachfolgend in dieser Bedeutung verwendet. Entsprechendes gilt für die vorliegend als 4- bzw. 5-Chlorogensäure bezeichneten Isomeren.

Der Erfindung liegt demgemäß die Aufgabe zugrunde, ein vereinfachtes Verfahren zur Abtrennung und Gewinnung von freier Chlorogensäure oder von Salzen derselben zu entwickeln, bei welchem keine toxikologisch bedenklichen Hilfschemikalien erforderlich sind. Darüber hinaus soll das Verfahren gewünschtenfalls die Auftrennung in die drei Isomeren ermöglichen.

Gegenstand der Erfindung ist ein Verfahren zur Abtrennung und Gewinnung von Chlorogensäure durch Extraktion von geeigneten (d.h. eine ausreichende Menge an Chlorogensäure enthaltenden) pflanzlichen Rohstoffen und Aufarbeitung des Extraktes, welches dadurch gekennzeichnet ist, daß man die Chlorogensäure mit Hilfe der Gelpermeations-Chromatographie an einem Molekularsieb aus einem vernetzten modifizierten Polysaccharid und insbesondere einem vernetzten Dextran aus dem Extrakt abtrennt und die Chlorogensäure und/oder den von Chlorogensäure befreiten Extrakt gewinnt.

Die Gelpermeations-Chromatographie an vernetzten modifizierten Polysacchariden dient im allgemeinen der Auftrennung von Substanzgemischen nach der Molekülgröße, d.h. die Moleküle erscheinen im Eluat in der Reihenfolge abnehmender Molekülgröße. Bei einer derartigen Auftrennung eines wässrigen, Chlorogensäure enthaltenden Pflanzenextraktes war demgemäß zu erwarten, daß die Chlorogensäure etwa in der Mitte des Elutionsspektrums und gemeinsam mit einer Reihe von Substanzen mit gleichem oder ähnlichem Molekulargewicht bzw. Molekülgröße erscheinen würde. Überraschenderweise zeigte es sich jedoch, daß Chlorogensäure sich auf einem Molekularsieb aus insbesondere Dextrangel nicht ihrem Molekulargewicht entsprechend verhält. Erfindungsgemäß wurde festgestellt, daß vernetzte modifizierte Polysaccharide und insbesondere Dextrane ein selektives Rückhaltevermögen für Chlorogensäure aufweisen, d.h., Chlorogensäure wird wesentlich länger festgehalten, als für deren Molekülgröße zu erwarten wäre.

Unerwarteterweise verläßt die Gesamtmenge der in dem Pflanzenextrakt enthaltenen Substanzen mit größerem und kleinerem Molekulargewicht als Chlorogensäure in einer breiten Fraktion die Trennsäule und die Chlorogensäure wird selektiv zurückgehalten. Sie ist durch weiteres Eluieren in einer relativ reinen und sauber abgetrennten Fraktion erhältlich.

Ein besonders geeignetes Material zur Auftrennung sind Gele von vernetzten Dextranen, wie sie

2

beispielsweise unter der Bezeichnung SEPHADEX® vertrieben werden. Erfindungsgemäß zeigte es sich ferner, daß die Trennschärfe mit steigendem Vernetzungsgrad des verwendeten Dextrangels ansteigt; d.h., bei vorgegebener Säulenlänge wird mit höher vernetztem Dextrangel eine schärfere Trennung erreicht als bei Verwendung eines Gels mit niedrigerem Vernetzungsgrad. Als Hinweis auf den Vernetzungsgrad eines Gels kann dessen Quellfähigkeit dienen. Letzere sinkt mit steigendem Vernetzungsgrad. So werden beispielsweise zur Herstellung von 100 ml gequollenem Gel 20 g Trockenmaterial von SEPHADEX® G 25 benötigt, während zur Herstellung des gleichen Gelvolumens 40 g des erheblich höher vernetzten SEPHADEX® G 10 erforderlich sind.

Die geeignete Arbeitstemperatur für die Auftrennung liegt im Bereich von etwa 10 bis 80°C, wobei normalerweise Raumtemperatur bevorzugt ist. Üblicherweise werden wässrige Pflanzenextrakte für die Auftrennung verwendet. Das Elutionsmedium kann somit ausschließlich aus Wasser, aber auch einem Gemisch von Wasser mit beispielsweise Alkoholen bestehen. Durch die Wahl des Lösungsmittelgemisches läßt sich die jeweils gewünschte Polarität des Lösungsmittelsystems beeinflussen.

Erfindungsgemäß umfaßt der Begriff "Chlorogensäure" sowohl die freie Säure als auch deren Salze. Bei pH-Werten, wie sie sich in wässrigen Pflanzenextrakten einstellen, dürfte die Chlorogensäure überwiegend in Salzform vorliegen. So beträgt der pH-Wert eines wässrigen Heidelbeerblätter-Extrakts ca. 3,8 und derjenige eines Rohkaffee-Extrakts etwa 5,5.

Soll im wesentlichen freie Chlorogensäure nach dem erfindungsgemäßen Verfahren hergestellt werden, so muß der pH-Wert des Extrakts entsprechend gesenkt werden. Es wurde festgestellt, daß sich freie Chlorogensäure durch vernetztes Dextran leicht und in verhältnismäßig reiner Form abtrennen läßt, wenn man den pH-Wert des Pflanzenextraktes auf etwa 2 bis 2,8 bringt. Es ist dabei nicht wesentlich, welche Säure zur Erniedrigung des pH-Wertes zugesetzt wird. Mineralsäuren wie Salzsäure und Schwefelsäure haben sich als besonders geeignet erwiesen. Bei bestimmten Aufgabenstellungen kann es günstiger sein, den erforderlichen sauren pH-Wert ohne Säurezusatz einzustellen. Dies kann dadurch erreicht werden, daß der Extraktlösung mit einem Ionenaustauscher die Kationen entzogen werden.

Eine besonders gute Auftrennung wird erreicht, wenn man mindestens zwei Dextrangele von unterschiedlichem Vernetzungsgrad verwendet und den die Chlorogensäure enthaltenden Teil des Eluats des ersten Gels anschließend auf das zweite Gel gibt. Man verwendet dabei für die erste Auftrennung das Gel mit dem niedrigeren Vernetzungsgrad. Die stärker vernetzte Dextranmatrix mit einem niedrigen Quellungsgrad (z.B. SEPHADEX® G 15) hat erfindungsgemäß ein besseres Auflösungsvermögen als eine weniger stark vernetzte Type (z.B. SEPHADEX® G 25). Allerdings sind dies für die praktische Anwendung nicht die allein entscheidenden Größen. So erfordert das Auftrennen viskoser Lösungen ein Gel mit günstigen Durchflußeigenschaften, wofür ein Dextran mit niedrigerem Vernetzungsgrad (wie SEPHADEX® G 25 coarse) gut geeignet ist.

Es wurde weiterhin festgestellt, daß sich mit Hilfe des erfindungsgemäßen Verfahrens gemäß der oben genannten Ausführungsform, d.h. ebenfalls bei etwa pH 2, auch die Isomeren der Chlorogensäure auftrennen lassen, wenn man eine ausreichend lange Molekularsieb-Säule verwendet, da dann die isomeren 5-, 4- und 3-Chlorogensäuren in dem Eluat nacheinander erscheinen und getrennt aufgefangen werden können. Es lassen sich auf diese Weise weitgehend reine Präparate der einzelnen Isomeren gewinnen.

Soll die Chlorogensäure in Salzform nach dem erfindungsgemäßen Verfahren abgetrennt und gewonnen werden, so kann bei dem natürlichen pH-Wert des wässrigen Pflanzenextraktes gearbeitet werden. Dies hat den Vorteil, daß die von dem Chlorogensäuresalz befreiten Extrakte unverändert bleiben und im Lebensmittelbereich weiter verwendet werden können.

Allerdings wird bei dieser Ausführungsform des erfindungsgemäßen Verfahrens eine geringere Trennschärfe erzielt, als dies bei der Trennung und Gewinnung freier Chlorogensäure aus einem angesäuerten Extrakt der Fall ist. Demgemäß kommt bei dieser Ausführungsform der erfindungsgemäß festgestellten Verbesserung der Trennschärfe mit steigendem Vernetzungsgrad des verwendeten Gels erhöhte Bedeutung zu.

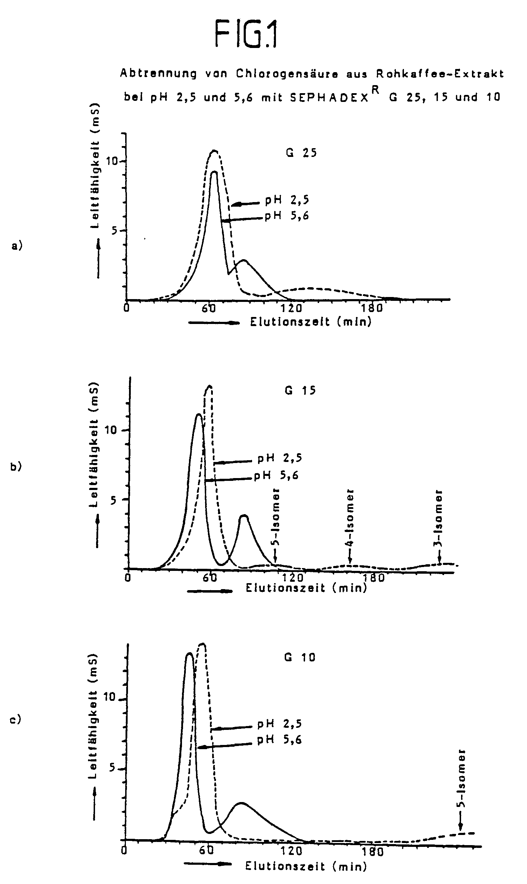

Wie in Figur 1(a) bis (c) dargestellt, ist die Trennschärfe bei vorgegebener Säulenlänge sowohl eine Funktion des pH-Wertes des aufzutrennenden Pflanzenextraktes als auch des Vernetzungsgrades des verwendeten Dextrangels. Aus entcoffeiniertem Rohkaffee-Extrakt wurde die Chlorogensäure jeweils sowohl ohne Ansäuern des Extraktes bei dem natürlichen pH-Wert von 5,6 als auch nach Ansäuern bei pH 2,5 an den SEPHADEX® Gelen G 25, G 15 und G 10 abgetrennt, wobei G 25 (Figur 1(a)) den niedrigsten und G 10 (Figur 1(c)) den höchsten Vernetzungsgrad aufweist. Es wurde ansonsten unter konstanten Bedingungen gearbeitet, wobei das Gelvolumen 150 ml, die Fließgeschwindigkeit 150 ml/h und die Temperatur 25°C betrug. Das Eluat wurde mit einem Leitfähigkeitsdetektor kontrolliert. Es wurden jeweils 20 ml eines Extrakts mit einer Trockenstoffkonzentration von 20 g/100 ml auf die Säulen aufgetragen. Wie Figur 1(a) zeigt, wird bei einem pH-Wert von 2,5 bereits mit dem niedrig vernetzten SEPHADEX® G 25 eine deutliche Abtrennung der Chlorogensäure erhalten, während bei pH 5,6 die Peaks noch stark überlappen. Demgegenüber wird bei Verwendung des höher vernetzten Gels G 15 auch bei dem nicht angesäuerten Extrakt eine nahezu vollständige Abtrennung der Chlorogensäure von den übrigen Bestandteilen erreicht (vgl. Figur 1(b)). Bei Verwendung von SEPHADEX® G 10 (vgl. Figur 1(c)) tritt auch bei dem nicht angesäuerten Extrakt durch die beginnende Differenzierung der einzelnen Isomeren eine verbreiterte Basis des Chlorogensäurepeaks auf. Bei pH-Werten oberhalb von 2,8, wie sie sich gewöhnlich bei der Herstellung

EP 0 258 297 B1

wässriger Pflanzenextrakte einstellen, werden demgemäß vorzugsweise höher vernetzte Gele wie beispielsweise SEPHADEX® G 15 und noch höher vernetzte Gele eingesetzt.

Selbstverständlich kann die Trennschärfe bei der Gewinnung von Chlorogensäure-Salzen aus Pflanzenextrakten mit pH-Werten oberhalb von 2,8 auch bzw. zusätzlich durch eine Steigerung der Säulenlänge erreicht werden.

Die Chlorogensäure bzw. deren Isomere können in den einzelnen Eluat-Fraktionen durch Hochdruckflüssigkeitschromatographie (HPLC) analysiert werden. Auf diese Weise kann für ein bestimmtes Säulenmaterial und eine gegebene Säulenlänge festgestellt werden, welche Eluatfraktionen die Chlorogensäure enthalten und inwieweit die Begleitstoffe enthaltende Vorlauf abzutrennen ist. Aus den die Chlorogensäure enthaltenden Eluatfraktionen kann erstere auf übliche Weise, besonders schonend beispielsweise durch Gefriertrocknung isoliert und als Festsubstanz gewonnen werden. Die die Begleitstoffe enthaltenden Fraktionen können entsprechend behandelt werden, um einen von Chlorogensäure freien Pflanzenextrakt zu gewinnen.

Als Ausgangsmaterial eignen sich pflanzliche Rohstoffe, welche Chlorogensäure in ausreichender Konzentration enthalten. In der nachfolgenden Tabelle sind die Chlorogensäuregehalte verschiedener Pflanzenmaterialien zusammengestellt, wobei die quantitative Analyse anhand HPLC durchgeführt wurde.

TABELLE 1

| Pflanzenmaterial | Chlorogensäure (% i.Tr.) |
|---|---|
| Grüne Kaffeebohnen | 5,0 |
| Heidelbeerblätter | 3,8 |
| Maté-Tee | 3,7 |
| Holunder-Blätter | 2,4 |
| Virginina-Tabak-Blätter | 1,4 |
| Weißdorn-Blätter | 0,6 |
| Ahorn-Blätter | 0,3 |
| Geißblatt-Blätter | 1,4 |
| Geißblatt-Früchte | 5,0 |
| Mahonia-Früchte | 1,6 |
| Apfel — unreife Früchte | 0,5 |

Gegenüber den bislang bekannten Verfahren bietet das erfindungsgemäße eine Reihe von Vorteilen. Das Verfahren ist wesentlich einfacher, da es weniger Verfahrensschritte und nur geringen apparativen Aufwand erfordert. Besonders wesentlich ist, daß die im pflanzlichen Rohstoff enthaltene Chlorogensäure weitgehend vollständig isoliert und gewonnen werden kann. Wichtig ist ferner, daß das Verfahren nicht auf coffeinhaltige Ausgangsmaterialien wie grüne Kaffeebohnen beschränkt ist, sondern daß auch andere preiswertere Rohstoffe eingesetzt werden können.

Es besteht die Möglichkeit, das Verfahren semikontinuierlich durchzuführen, da die Trenneigenschaften des Molekularsiebs offensichtlich sehr lange erhalten bleiben. So zeigte z.B. eine mit vernetztem Dextran gefüllte Säule auch nach über 100 Durchgängen unverändert reproduzierbare Eigenschaften. Ein Durchgang besteht jeweils aus

Auftragen des Extrakes,
Eluieren der Begleitstoffe,
Eluieren von Chlorogensäure und
Spülen.

Unmittelbar an das Spülen des Gels kann der nächste Durchgang angeschlossen werden. Bei Verschmutzung der Säule kann die Füllung mit einfachen, im Stand der Technik bekannten Mitteln gereinigt werden. Schwebstoffe, die den Durchlauf verschlechtern, können durch Aufrühren des Gels in Wasser abgeschwemmt werden. Vom Gel festgehaltene Farbstoffe können mit 0,2%-iger Natronlauge im Durchlauf entfernt werden, ohne daß dadurch das Trennverhalten beeinflußt wird. Zur näheren Erläuterung der Erfindung sollen die nachfolgenden Beispiele dienen.

4

### Beispiel 1

Gewinnung von freier Chlorogensäure aus Heidelbeerblättern

Zur Erzeugung von 175 ml Gel wurden 35 g vernetztes Dextran (SEPHADEX® G 25 coarse) mit einem Überschuß an Wasser über Nacht gequollen. Das so erhaltene Gel wurde in eine Säule überführt und zwischen zwei Adaptern fixiert.

50 g Heidelbeerblätter (Herkunftsland UdSSR) wurden mit 500 ml entmineralisiertem Wasser bei 80°C extrahiert. Der verdünnte Extrakt wurde filtriert und im Vakuum auf 25 ml eingeengt. Er enthielt 1,1 g Chlorogensäure und 9,0 g andere Extraktbestandteile. Mit 1,25 ml 37%-iger Salzsäure wurde der pH-Wert des Extraktes von 3,8 auf 2,0 verschoben.

Die Lösung wurde dann mit einer Schlauchpumpe auf die Trennsäule gegeben und nachfolgend mit Wasser bei einem konstanten Durchfluß von 100 ml/Std. eluiert. Mit einem Fraktionssammler wurde das Eluat in 25 ml Fraktionen getrennt.

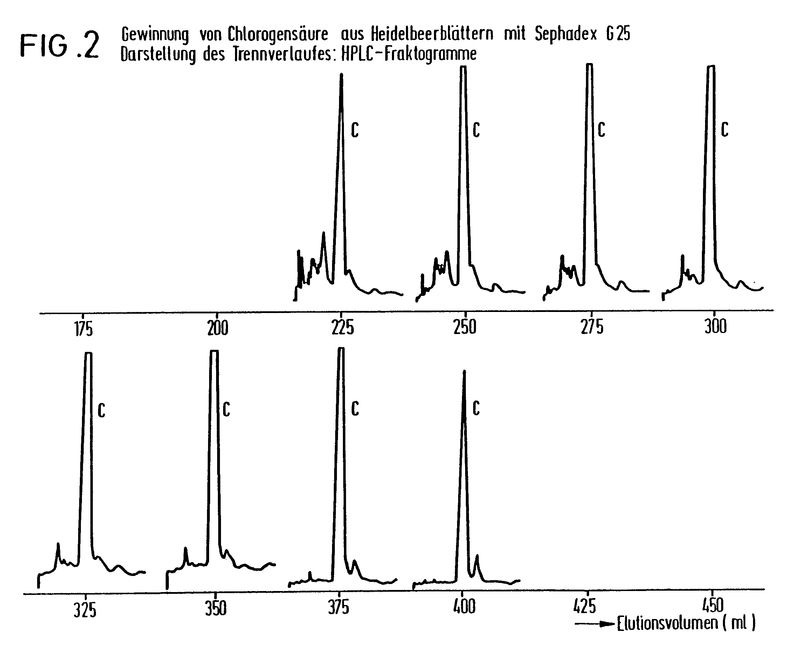

In ihnen wurde mit HPLC der Chlorogensäuregehalt bestimmt. Figur 2 zeigt die HPLC-Fraktogramme in Abhängigkeit vom Elutionsvolumen. Es ist zu erkennen, daß die Fraktionen bis zu einem Elutionsvolumen von 200 ml keine Chlorogensäure enthalten.

Unter den gewählten Trennbedingungen befindet sich die Chlorogensäure im Elutionsvolumen zwischen 200 und 425 ml. Die ausgewählten Fraktionen wurden vereinigt, im Vakuum eingeengt und danach gefriergetrocknet. In den erhaltenen Trockensubstanzen wurde der Gehalt an Chlorogensäure nach Auflösen definierter Mengen in Wasser im Vergleich zu einem Standard mit HPLC bestimmt.

In der nachfolgenden Tabelle 2 sind die erhaltenen Trockensubstanz-Mengen und die darin ermittelten Chlorogensäure-Gehalte angegeben.

### TABELLE 2

| Elutionsvolumen (ml) | Trockensubstanz (mg) | Chlorogensäure (%) |
|---|---|---|
| 200—250 | 670 | 40,9 |
| 250—300 | 575 | 64,0 |
| 300—350 | 345 | 78,1 |

Daraus ist zu entnehmen, daß die Präparate noch in unterschiedlichem Maße mit anderen Substanzen verunreinigt sind. Das ist auch bei der Betrachtung der HPLC-Peakbilder zu erkennen.

Die Chlorogensäure enthaltenden Trockensubstanzen wurden wieder in 25 ml Wasser aufgelöst, wobei sich ein pH-Wert von 2,3 ergab und mit 150 ml Gel (SEPHADEX® G 15) gereinigt. Die Vorgehensweise war analog der vorher beschriebenen Trennung. Bei der zweiten Säule wurde die Chlorogensäure im Elutionsvolumen zwischen 650 und 1150 ml erhalten. Auch hier wurden ausgewählte Fraktionen zusammengefaßt, im Vakuum konzentriert und danach gefriergetrocknet. Die Trockenpräparate wurden wie vorher beschrieben auf ihren Reinheitsgrad untersucht. Die Ergebnisse zeigt Tabelle 3.

### TABELLE 3

| Elutionsvolumen (ml) | Trockensubstanz (mg) | Chlorogensäure (%) |
|---|---|---|
| 650—775 | 400 | 81,5 |
| 775—900 | 300 | 94,2 |
| 900—1025 | 210 | 92,2 |
| 1025—1150 | 85 | 85,0 |

Aus den Zahlen geht hervor, daß die Chlorogensäure ohne weiteres Umkristallisieren schon mit einem Reinheitsgrad von über 94% erhalten wurde.

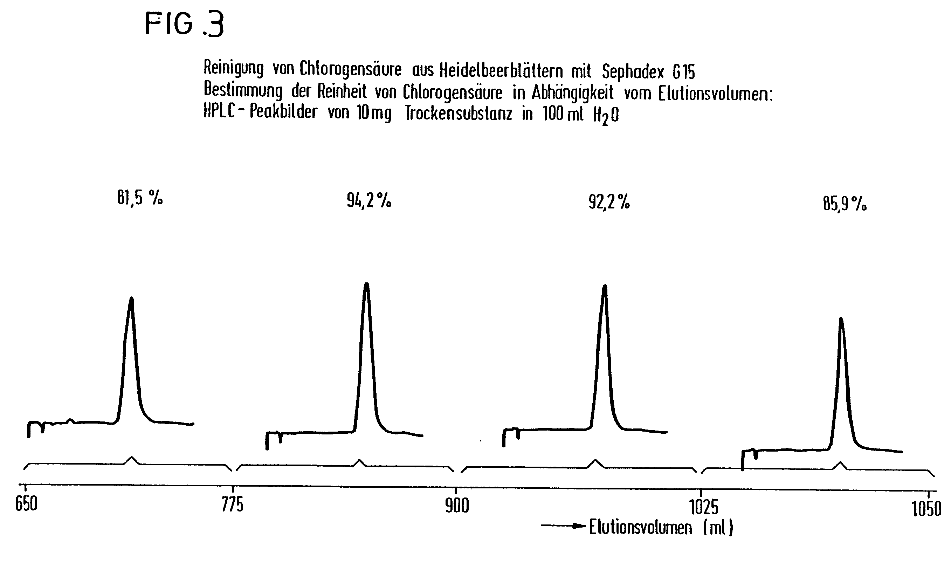

Figur 3 zeigt die HPLC-Fraktogramme der hergestellten Trockenpräparate aus verschiedenen Elutionsvolumina. Die HPLC-Peakbilder veranschaulichen die erzielte Reinheit der so hergestellten Chlorogensäure-Präparate.

### Beispiel 2

Aufarbeitungsvarianten

Es besteht die Möglichkeit, mit entsprechend großen Säulen gleich im ersten Schritt zu höheren Reinheitsgraden zu gelangen, doch ist von Fall zu Fall zu entscheiden, welche Vorgehensweise sinnvoll ist. Entscheidend ist der Gehalt an Chlorogensäure im Ausgangsmaterial. Ist der Anteil der Chlorogensäure im Trockenextrakt kleiner als 10%, so ist es sinnvoller, mit zwei Trennsäulen zu arbeiten. Es ist hierbei nicht erforderlich, die Chlorogensäure-Lösung zwischen den beiden Säulenpassagen aufzukonzentrieren. Am effektivsten wird wie folgt vorgegangen: Der Pflanzenextrakt wird als ca. 25%-ige Lösung durch die erste Säule geführt (z.B. SEPHADEX® G 25 coarse). Das zuerst austretende Eluat mit den Begleitsubstanzen wird ausgeblendet. Erst wenn die Chlorogensäure das Ende der ersten Säule erreicht hat, wird das Eluat direkt in die zweite Säule (z.B. SEPHADEX® G 15) geleitet. Durch diese Vorgehensweise wird die zur Reinigung der Chlorogensäure vorgesehene zweite Säule nicht mit dem Hauptteil der Begleitsubstanzen belastet.

Beispiel 3

Trennung eines Chlorogensäure-Isomerengemisches

Für diesen Versuch wurden zwei Trennsäulen hintereinander geschaltet — eine Säule mit 175 ml SEPHADEX® G 25 coarse und eine mit 150 ml SEPHADEX® G 15. Es wurden 1,6 g Chlorogensäure-Isomerengemisch in 10 ml $H_2O$ bei 40°C gelöst und die saure Lösung wurde mit einer Schlauchpumpe als Startzone in das Trennsystem gedrückt. Eluiert wurde mit Wasser bei einer Durchflußgeschwindigkeit von 75 ml/Std. Das austretende Eluat wurde 25 ml-weise fraktioniert und mit Hilfe von HPLC auf Chlorogensäure analysiert.

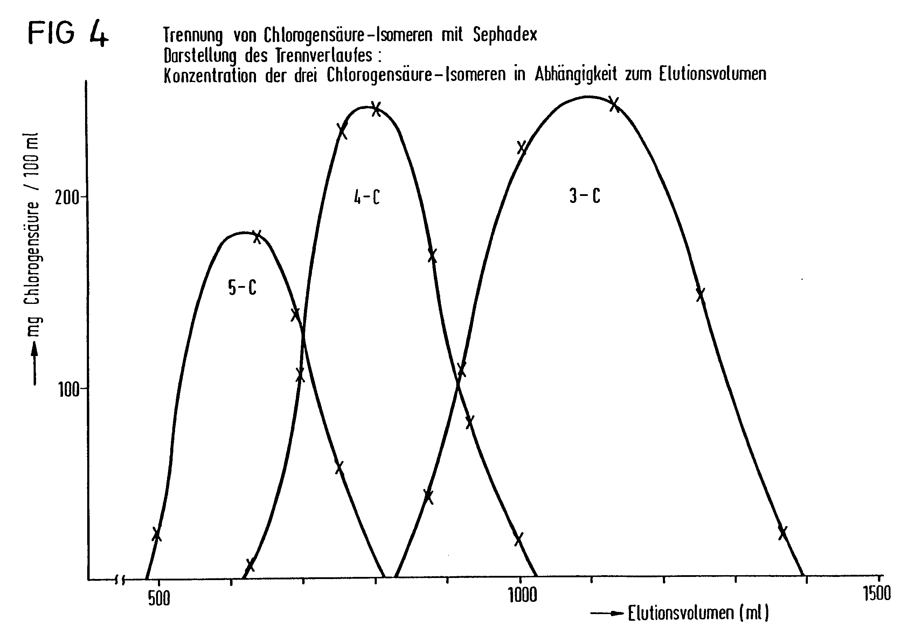

In Figur 4 sind die Konzentrationen der Chlorogensäure in Abhängigkeit vom Elutionsvolumen dargestellt. Es ist eine deutliche Trennung in die drei Chlorogensäure-Isomeren zu erkennen. Der Abstand der Chlorogensäure-Isomeren kann durch Verlängerung der Trennsäule weiter auseinander gezogen werden.

Beispiel 4

Gewinnung von Chlorogensäure in Salzform aus Rohkaffee ohne pH-Korrektur

100 g entcoffeinierter Roh-Kaffee (Herkunftsland Columbien) wurden mit 300 ml entmineralisiertem Wasser bei 80°C extrahiert. Der erhaltene Extrakt wies einen pH-Wert von 5,6 auf. Die Lösung wurde anschließend auf 50 ml eingeengt. Die Extraktkonzentration betrug 22,5 g Trockensubstanz/100 ml. 20 ml dieser Lösungen wurden mit einer Schlauchpumpe in eine Säule mit 150 ml Gel (SEPHADEX® G 15) gefördert.

Die Säule wurde mit Wasser bei einer Temperatur von 65°C und einer Fließgeschwindigkeit von 300 ml/h eluiert. Das Eluat wurde in Fraktionen von jeweils 25 ml aufgefangen und der Chlorogensäuregehalt der Fraktionen mit HPLC bestimmt.

Die Ergebnisse sind in Tabelle 4 wiedergegeben.

Es zeigte sich, daß die Fraktionen bis zu einem Elutionsvolumen von 150 ml keine Chlorogensäure enthalten. In dem Elutionsvolumen zwischen 150 und 225 ml wurde Chlorogensäure (überwiegend als K-Salz) bereits im ersten Durchgang mit einem Reinheitsgrad von 70,7% erhalten.

TABELLE 4

| Elutionsvolumen (ml) | Trockensubstanz (g) | Chlorogensäure (%) |
|---|---|---|
| 25—150 | 3,17 | 0 |
| 150—225 | 1,30 | 70,7 |

Die Ausbeute betrug 97,9% bezogen auf den Chlorogensäuregehalt der eingesetzten Trockensubstanz.

**Patentansprüche**

1. Verfahren zur Abtrennung und Gewinnung von Chlorogensäure durch Extraktion von pflanzlichen Rohstoffen und Aufarbeitung des gewonnenen Extraktes durch Säulenchromatographie, dadurch gekennzeichnet, daß man die Chlorogensäure mit Hilfe der Gelpermeations-Chromatographie an einem Molekularsieb aus einem vernetzten modifizierten Polysaccharid von den Begleitstoffen abtrennt und die Chlorogensäure und/oder den von Chlorogensäure befreiten Extrakt gewinnt.

2. Verfahren nach Anspruch 1, dadurch gekennzeichnet, daß man als Molekularsieb ein vernetztes Dextran verwendet.

3. Verfahren nach Anspruch 1 oder 2, dadurch gekennzeichnet, daß man zur Gewinnung von im wesentlichen freier Chlorogensäure den wässrigen Pflanzenextrakt auf einen pH-Wert im Bereich von etwa 2 bis 2,8 einstellt.

4. Verfahren nach Anspruch 3, dadurch gekennzeichnet, daß man die Gelpermeations-Chromatographie an mindestens zwei Dextrangelen von unterschiedlichem Vernetzungsgrad durchführt, wobei das erste Gel den niedrigeren Vernetzungsgrad besitzt, und den die Chlorogensäure enthaltenden Teil des Eluats des ersten Gels anschließend auf das zweite Gel gibt.

5. Weitere Ausbildung des Verfahrens nach Anspruch 3 oder 4 zur Gewinnung der isomeren 5-, 4- und 3-Chlorogensäuren, dadurch gekennzeichnet, daß man bei der Durchführung der Gelpermeations-Chromatographie in der letzten Stufe eine ausreichend lange Säule einsetzt, aus welcher die isomeren 5-, 4- und 3-Chlorogensäuren nacheinander eluiert und die Eluate getrennt aufgefangen werden.

## Revendications

1. Procédé de séparation et d'obtention d'acide chlorogénique par extraction de matières premières végétales et traitement de l'extrait obtenu par chromatographie sur colonne, caractérisé en ce que l'acide chlorogénique est séparé des matières d'accompagnement par chromatographie par perméation de gel sur un tamis moléculaire à partir d'un polysaccharide modifié réticulé, et l'acide chlorogénique et/ou l'extrait libéré de l'acide chlorogénique est séparé.

2. Procédé selon la revendication 1, caractérisé en ce qu'on utilise comme tamis moléculaire un dextrane réticulé.

3. Procédé selon l'une des revendications 1 et 2, caractérisé en ce que pour extraire l'acide chlorogénique essentiellement libre, on règle l'extrait végétal aqueux à une valeur de pH comprise entre sensiblement 2 et 2,8.

4. Procédé selon la revendication 3, caractérisé en ce que l'on exécute la chromatographie par perméation de gel sur au moins deux gels de dextrane de degrés différents de réticulation, le premier gel possédant le degré de réticulation le plus faible, et en ce que l'on transmet immédiatement au second gel la partie de l'éluat du premier gel contenant l'acide chlorogénique.

5. Procédé selon la revendication 3 ou 4 pour l'obtention des isomères 5, 4 et 3 de l'acide chlorogénique, caractérisé en ce que pendant l'exécution de la chromatographie par perméation de gel, on introduit une colonne suffisamment longue dans le dernier étage, à partir de laquelle les isomères 5, 4 et 3 de l'acide chlorogénique sont élués l'un après l'autre, et les éluats sont recueillis séparément.

## Claims

1. Process for separation and production of chlorogenic acid by extracting plant raw materials and processing the resulting extract by column chromatography, characterized in that the chlorogenic acid is separated from the accompanying substances by gel permeation chromatography on a molecular sieve of a cross-linked modified polysaccharide and the chlorogenic acid and/or the extract freed from chlorogenic acid are obtained.

2. Process according to claim 1, characterized in that a cross-linked dextran is used as a molecular sieve.

3. Process according to claims 1 or 2, characterized in that to obtain substantially free chlorogenic acid the aqueous plant extract is adjusted to a pH in the range of about 2 to about 2.8.

4. Process according to claim 3, characterized in that gel permeation chromatography is performed on at least two dextran gels of different degrees of cross-linkage, the first gel having the lower degree of cross-linkage and that the chlorogenic acid containing part of the eluate of the first gel then being fed onto the second gel.

5. Further embodiment of the process according to claims 3 or 4 for the preparation of isomeric 5-, 4- and 3-chlorogenic acids, characterized in that when gel permeation chromatography is performed, in the last stage a column of sufficient length is used, from which the isomeric 5-, 4- and 3-chlorogenic acids are eluated in succession and the eluates are gathered separately.

EP 0 258 297 B1

# FIG.1

Abtrennung von Chlorogensäure aus Rohkaffee-Extrakt
bei pH 2,5 und 5,6 mit SEPHADEX[R] G 25, 15 und 10

1

FIG .2   Gewinnung von Chlorogensäure aus Heidelbeerblättern mit Sephadex G 25
Darstellung des Trennverlaufes: HPLC-Fraktogramme

Elutionsvolumen ( ml )

EP 0 258 297 B1

# FIG .3

Reinigung von Chlorogensäure aus Heidelbeerblättern mit Sephadex G 15
Bestimmung der Reinheit von Chlorogensäure in Abhängigkeit vom Elutionsvolumen:
HPLC - Peakbilder von 10 mg Trockensubstanz in 100 ml $H_2O$

81,5 %          94,2 %          92,2 %          85,9 %

650          775          900          1025          1050

Elutionsvolumen (ml)

EP 0 258 297 B1

FIG 4  Trennung von Chlorogensäure-Isomeren mit Sephadex
Darstellung des Trennverlaufes :
Konzentration der drei Chlorogensäure-Isomeren in Abhängigkeit zum Elutionsvolumen

EP 0 258 297 B1